# FASCICULE DE BREVET EUROPEEN

(11) **EP 0 682 112 B1**
(45) Date de publication et mention de la délivrance du brevet: **11.04.2007**
(21) Numéro de dépôt: 95107952.4
(22) Date de dépôt: 19.03.1990
(51) Int. Cl.: C12N 15/00, C12N 15/86, C12N 15/90, A01K 67/027, A61K 48/00

(54) **Procédé pour obtenir l'expression, ou pour améliorer le taux d'expression, d'un gène**
Verfahren zur Erreichung einer Expression, oder zur Verbesserung der Expression eines Gens
Method for archiving expression, or to improve the level of expression of a gene

(30) Priorité: 20.03.1989 FR 8903630
(43) Date de publication de la demande: 15.11.1995
(62) Demande divisionnaire de: 90905207.8
(73) Titulaire: INSTITUT PASTEUR, 75724 Paris Cédex 15 (FR)
(72) Inventeur: LE MOUELLIC, Hervé, F-75009 Paris (FR); BRULET, Philippe, F-75015 Paris (FR)
(74) Mandataire: Almond-Martin, Carol

(56) Documents cités:
- EP-A- 0 074 808
- EP-A- 0 279 582
- CELL, vol. 56, no. 2, 27 Janvier 1989 CELL PRESS,CAMBRIDGE,MA,US;, pages 145-147, M.A. FROHMAN AND G.R. MARTIN 'Cut, Paste, and Save: New approaches to altering specific genes in mice'
- CELL, vol. 56, no. 2, 27 Janvier 1989 CELL PRESS,CAMBRIDGE,MA,US;, pages 313-321, S. THOMPSON ET AL. 'Germ line transmission an dexpression of a corrected HPRT gene produced by gene targeting in embryonic stem cells'
- NATURE, vol. 336, 24 Novembre 1988 MACMILLAN JOURNALS LTD., LONDON,UK, pages 348-352, S.L. MANSOUR ET AL. 'Disruption of the proto-oncogene int-2 in mouse embryo-derived stem cells: a general strategy for targeting mutations to non-selectable genes'
- PROC. NATL.ACAD SCI., vol. 85, no. 22, Novembre 1988 NATL. ACAD SCI.,WASHINGTON,DC,US;, pages 8583-8587, T. DOETSCHMAN ET AL. 'Targeted mutation of the Hprt gene in mouse embryonic stem cells'
- NATURE, vol. 338, 9 Mars 1989 MACMILLAN JOURNALS LTD., LONDON,UK, pages 150-153, A. ZIMMER AND P. GRUSS 'Production of chimaeric mice containing embryonic stem (ES) cells carrying a homeobox Hox1.1 alleele mutated by homologous recombination'
- SCIENCE, vol. 245, 15 Septembre 1989 AAAS,WASHINGTON,DC,US, pages 1234-1236, R.S. JOHNSON ET AL. 'Targeting of nonexpressed genes in embryonic stem cells via homologous recombination'

## Description

L'invention concerne un procédé de remplacement spécifique d'une copie d'un gène présent dans le génome d'un organisme eucaryote receveur par l'intégration d'un gène différent du gène inactivé. De préférence, le gène receveur sera présent en au moins 2 exemplaires dans la cellule hôte transfectée. Le gène receveur est défini comme étant le gène ou se fera l'insertion du gène différent.

L'invention concerne un procédé pour obtenir l'expression, ou pour améliorer le taux d'expression d'un gène, dit gène receveur, dans le génome d'une cellule eucaryote, ledit gène receveur étant susceptible d'être rendu fonctionnel ou plus efficace lorsqu'il est recombiné avec un ADN de complément, ledit procédé comprenant :
- l'introduction dans des cellules eucaryotes qui ne sont ni des cellules germinales humaines, ni des cellules embryonnaires humaines, d'un vecteur contenant un insérat comprenant lui-même l'ADN de complément, dit ADN d'insertion, et deux séquences dites "flanquantes" de part et d'autre de l'ADN d'insertion, respectivement homologues à deux séquences génomiques qui jouxtent le site d'insertion souhaité dans le gène receveur ;
- l'ADN d'insertion comportant une partie d'un gène, et étant hétérologue vis-à-vis du gène receveur ;
- les séquences flanquantes étant choisies afin de permettre par recombinaison homologue, la juxtaposition de l'ADN d'insertion dans le gène receveur, de façon à ce que l'ADN d'insertion rende fonctionnel ou améliore le fonctionnement du gène receveur.

Plus particulièrement, l'invention concerne la production d'animaux transgéniques non-humains dans lesquels le gène étranger a été introduit d'une manière ciblée pour permettre, à la fois, le maintien des fonctions génétiques normales de l'animal et l'expression du gène étranger sous le contrôle de promoteurs endogènes.

Par "gène différent ou étranger" on entend toute séquence nucléotidique correspondant à la totalité ou à une partie d'un gène "étranger ou différent" du gène récepteur telle qu'elle est trouvée normalement dans le génome (ARN ou ADN), ou elle correspond également à une séquence modifiée artificiellement du gène normal ou encore à un fragment de cette séquence.

L'invention concerne également le procédé de production de ces animaux transgéniques non humains.

Dans la production d'animaux transgéniques, les méthodes conventionnelles utilisées pour l'introduction de séquences d'ADN hétérologues dans la lignée cellulaire germinale, ne permettent pas de contrôler le site de l'intégration du gène étranger dans le génome, ni le nombre de copies ainsi introduit. L'intégration du gène étranger se fait au hasard et, en général, plusieurs copies du gène s'intègrent en même temps, parfois sous forme de tandem tête à queue, le site de l'intégration et le nombre de copies intégrées variant d'un animal transgénique à l'autre.

Il peut donc arriver que des gènes cellulaires endogènes, situés au point d'insertion, soient ainsi inactivés, sans que cela soit facilement décélable en raison de nombreuses insertions au hasard. Si le produit de ces gènes est important pour le développement de l'animal, celui-ci sera sérieusement perturbé. D'ailleurs, l'insertion aléatoire du gène étranger peut se faire à un site qui n'est pas approprié pour l'expression du gène. De plus, le fait qu'il y ait variation du site et du nombre d'insertions d'animal en animal rend l'interpétation des études d'expression extrêmement difficile.

Un problème majeur rencontré dans la production d'animaux transgéniques, est l'obtention de l'expression du gène étranger. D'une manière générale, deux types d'expérience ont été réalisés chez les souris.

Les gènes introduits dans la lignée germinale sont :
- soit des gènes "complets", comprenant des séquences codantes flanquées de leurs propres séquences régulatrices;
- soit des gènes composites, formés de la séquence codante d'un gène fusionnée à la séquence promotrice d'un autre gène, les deux fragments appartenant même parfois à deux espèces animales différentes.

On a pu ainsi confirmer que la spécificité de l'expression des gènes dans tel ou tel tissu est déterminée par leur(s) séquence(s) régulatrices.

Le choix du promoteur approprié pour l'expression du gène étranger chez l'animal transgénique est donc d'une importance primordiale.

D'autre part, la mutagénèse dirigée de gènes murins dans des cellules souches embryonnaires a récemment été réalisée en faisant appel à une technique de "ciblage génétique" (gene targeting) (Thomas et al., 1987; Thompson et al., 1989).

Dans le premier cas, le gène murin HPRT a été muté par insertion et remplacement et, dans le deuxième cas, un gène HPRT muté a été corrigé. Thomson et al. ont étendu leurs expériences jusqu'à l'obtention de souris chimères et ont constaté le passage de la modification génétique dans la lignée cellulaire germinale.

Dans chacun des documents cités, le site précis de l'intégration a été ciblé par recombinaison homologue entre, d'une part, des séquences exogènes comportant la mutation ou correction incluses dans un vecteur, sous le contrôle d'un promoteur exogène, et, d'autre part, leur homologue génomique. Ceci étant, il faut remarquer que les auteurs antérieurs ont réalisé leurs expériences sur un gène spécifique (HPRT) dont l'activation par mutation s'accompagnait d'un phénotype décelable. La mutation ciblée décrite par Thomas et al., avait pour effet d'inactiver le gène HPRT et, par conséquent, de faire disparaître le phénotype décelable normalement associé avec le HPRT. Le gène de sélection Neo^{R}, sous le contrôle d'un promoteur TK, était donc incorporé dans l'ADN d'insertion afin de permettre la sélection des transformants. Il est à noter que les expériences décrites dans l'art antérieur impliquaient une sélection soit par le gène receveur (p. ex HPRT) soit par le gène d'insertion (p. ex Neo^{R}). Le site de l'insertion et/ou le type de gène inséré est donc limité à des gènes conférant un caractère sélectable, une sélection directe.

En outre, dans l'art antérieur, les séquences exogènes sur le vecteur servent donc à la fois à cibler le site d'intégration et à introduire la modification. Suite à la recombinaison homologue, le gène modifié se trouve toujours dans son environnement génétique normal.

Rappelons qu'un problème qui se pose au cours de la production d'animaux transgéniques est le danger d'inactiver un gène cellulaire endogène qui se trouve au point d'insertion du gène étranger.

Selon la fonction du produit du gène inactivé, une telle inactivation peut conduire à des désordres physiologiques ou morphologiques importants chez l'animal transgénique, ou pourrait même empêcher sa survie.

En revanche, l'inactivation d'un gène pourrait être considéré comme avantageux si le gène en question codait pour un récepteur de virus ou autre agent infectieux.

Les inventeurs ont étudié la possibilité d'éviter les inconvénients décrits plus haut, et associés, dans certains cas, à l'inactivation possible d'un ou plusieurs gènes cellulaires endogène de fonction importante au cours de la production d'animaux transgéniques non humains.

L'invention a pour objet un procédé de remplacement ou d'insertion spécifique d'un gène, notamment par ciblage d'un ADN, dit ADN d'insertion constitué par la totalité d'un gène, ou par une partie d'un gène, susceptible d'être rendu fonctionnel, ou dont le fonctionnement peut être rendu plus efficace, lorsqu'il est recombiné avec un ADN de complément pour alors fournir un gène recombinant complet dans le génome d'une cellule eucaryote qui n'est ni une cellule germinale humaine ni une cellule embryonnaire humaine, caractérisé en ce que
- le site d'insertion se trouve dans un gène choisi, dit gène receveur, et contenant l'ADN de complément, et en ce que
- l'on transfecte des cellules eucaryotes qui ne sont ni des cellules germinales humaines ni des cellules embryonnaires humaines avec un vecteur contenant un insert comprenant lui-même l'ADN d'insertion et deux séquences dites "flanquantes" de part et d'autre de l' ADN d'insertion, respectivement homologues à deux séquences génomiques qui jouxtent le site d'insertion souhaité dans le gène receveur,
- l'ADN d'insertion étant hétérologue vis-à-vis du gène receveur, et
- les séquences flanquantes, étant choisies parmi celles qui constituent le susdit ADN de complément et qui autorisent, par recombinaison homologue avec des séquences correspondantes du gène receveur, la reconstitution d'un gène recombinant complet dans le génome de la cellule eucaryote.

Selon le procédé de l'invention, ledit ADN. d'insertion peut contenir soit une séquence codante soit une séquence régulatrice, le procédé étant alors caractérisé en ce que
- le site d'insertion se trouve dans un gène choisi dit gène receveur et en ce que
- l'on transfecte des cellules eucaryotes avec un vecteur contenant un insérat comprenant lui-même l'ADN d'insertion et deux séquences dites "flanquantes" de part et d'autre de l'ADN d'insertion, respectivement homologues à deux séquences génomiques qui jouxtent le site d'insertion souhaité dans le gène receveur,
- l'ADN d'insertion étant hétérologue vis-à-vis du gène receveur et,
- les séquences flanquantes étant choisies afin de permettre par recombinaison homologue selon le cas, soit l'expression de la séquence codante de l'ADN d'insertion entier sous le contrôle des séquences régulatrices du gène receveur, soit l'expression d'une séquence codante du gène receveur sous le contrôle de séquences régulatrices de l'ADN d'insertion.

Par exemple, l'ADN d'insertion peut contenir une séquence codante dépourvue d'élément de régulation, notamment d'un promoteur qui lui est propre.

L'invention concerne aussi un procédé de production d'animaux transgéniques non humains, caractérisé en ce que des cellules E.S. non humaines sont transfectées dans les conditions sus-décrites et sélectionnées pour l'évènement de recombinaison homologue, à savoir l'intégration correcte du gène étranger, les cellules transfectées sont injectées dans des embryons non humains à un stade où ils sont aptes à intégrer les cellules transfectées (par exemple au stade blastocyste), ceux-ci sont ensuite réimplantés dans une mère porteuse non humain et les individus chimères non humains obtenus au terme de la gestation, et chez lesquels est constatée la colonisation par les cellules E.S. de la lignée germinale, sont accouplés. Si les cellules E.S. ont colonisé la lignée germinale de l'animal chimère non humain des animaux transgéniques hétérogozytes non humains pour le gène remplacé seront obtenus par accouplement (F1) dans la descendance.

Il est également possible d'insérer l'ADN d'insertion, porté par le vecteur de l'invention, dans l'oeuf non humain peu de temps après la fécondation (c'est-à-dire moins de 24 heures). De cette manière, l'insertion est effectuée pendant que l'oeuf non humain est à l'état unicellulaire.

L'invention concerne aussi un plasmide apte à effectuer l'insertion ciblée d'un gène recombinant dit gène d'insertion dans le génome d'une cellule eucaryote, caractérisé en ce qu'il contient un insérat comprenant lui-même le gène d'insertion et deux séquences dites "flanquantes" de part et d'autre du gène d'insertion, respectivement homologues aux deux séquences génomiques qui jouxtent le site d'insertion souhaité dans le gène receveur.

L'ADN d'insertion peut être un gène, ou une partie d'un gène, hétérologue à l'espèce transfectée.

L'invention concerne également des animaux transgéniques non humains dans lesquels au moins un gène endogène, qui de préférence est présent dans le - génome à au moins deux exemplaires, a été - inactivé par l'insertion d'un gène qui est différent du gène inactivé, le gène d'insertion étant inséré dans une position qui permet l'expression de ce gène sous le contrôle des séquences régulatrices du gène endogène inactivé.

Le procédé de l'invention permet, donc, grâce au phénomène de recombinaison homologue, d'insérer d'une manière ciblée des gènes étrangers , en particulier des séquences codantes dépourvues du promoteur qui leur est normalement associé, dans le génome d'un organisme eucaryote à un site qui permet leur expression sous le contrôle du promoteur endogène du gène où se fait l'insertion, et par conséquence, d'inactiver le gène endogène ciblé.

Selon un mode de réalisation préféré de l'invention, le gène receveur ciblé est un gène qui est présent dans le génome en au moins deux exemplaires. L'utilisation de la technique d'électroporation (Ref. 11) assure l'introduction d'une copie seulement du gène étranger.

Selon cette variante de l'invention, l'insertion ciblée du gène d'intérêt (c'est-à-dire le gène dit d'insertion) a pour effet d'inactiver la seule copie du gène cellulaire endogène où se fait l'insertion et laisse intacte et fonctionnelle la ou les autre(s) copie(s) de ce gène.

De cette façon, le fonctionnement génétique de l'animal transgénique non humain n'est pas ou peu perturbé par l'introduction du gène étranger, même si l'insertion inactive une seule copie d'un gène essentiel receveur pour le développement de l'animal. Soit son développement ne serait donc pas affecté par l'insertion du gène étranger, soit les perturbations mineures possibles dans le cas de l'inactivation d'un gène critique ne seraient probablement pas léthales pour l'animal non humain. Les effets de l'insertion du gène étranger à l'état homozygote pourraient être de toute nature et seraient observés en 2ème génération (F2) après croisements d'individus non humains hétérozygotes (F1) entre eux.

Si, par contre, l'inactivation de toutes les copies d'un gène est souhaitée, par exemple, dans le cas où le gène code pour un récepteur d'agent infectieux, de multiple copies du gène étranger sont introduites. Le contrôle de la quantité introduite peut être assuré en faisant appel à des techniques connues.

L'insertion ciblée du gène étranger permet donc son introduction dans un site où son expression est sous le contrôle des séquences régulatrices du gène endogène où se fait l'insertion.

Le procédé de l'invention permet ainsi d'insérer le gène étranger derrière un promoteur endogène qui a les fonctions désirées (par exemple, spécificité d'expression dans tel ou tel tissu), et cela, le cas échéant, sans inactiver les autres copies du gène receveur.

Selon un mode de réalisation préféré, l'ADN - d'insertion, ou le vecteur, contient des séquences intercalées entre l'ADN d'insertion et les séquences flanquantes. Ces séquences peuvent contenir par exemple une séquence codant pour un agent sélectif permettant la sélection des transformants et éventuellement un gène marqueur par exemple le LacZ.

Selon un mode de réalisation particulièrement préféré de l'invention, l'ADN d'insertion comporte entre les séquences flanquantes, d'une part une séquence d'ADN destinée à être recombinée avec l'ADN de complément dans le gène receveur pour fournir un gène recombinant, et, d'autre part, une séquence codant pour un agent sélectif permettant la sélection des transformants et un promoteur autorisant l'expression de l'agent sélectif, le gène receveur et le gène recombinant codant pour des produits d'expression ne conférant pas de phénotype sélectionable. Selon cette variante, l'insérât du plasmide de l'invention comprend, entre les séquences flanquantes, d'une part une séquence d'ADN destinée à être recombinée avec l'ADN de complément dans le gène receveur, et, d'autre part, une séquence codant pour un agent sélectif permettant la sélection des transformants et un promoteur autorisant l'expression de l'agent sélectif, la séquence d'ADN destinée à être recombinée avec l'ADN de complément étant autre qu'un gène codant pour un agent sélectif.

De cette manière, la sélection des transformants est entièrement indépendante de la nature du gène receveur et du gène inséré, contrairement aux procédés décrits jusqu'à ce jour dans lesquels le gène inséré ou le gène receveur devait par nécessité coder pour un produit d'expression permettant la sélection des transformants. Le système développé par les inventeurs permet une flexibilité totale en ce qui concerne la nature du gène receveur et du gène inséré ou du gène formé par la recombinaison homologue. Les inventeurs ont constaté d'une manière surprenante que l'insertion de séquences de taille importante (par exemple d'environ 7.5 kb) n'affecte pas la fréquence de recombinaison homologue.

L'effet que peut avoir l'insertion de la séquence d'ADN selon cet aspect de l'invention inclut, selon le type de séquence insérée par exemple le remplacement d'une séquence codante, le remplacement d'une séquence régulatrice, l'inactivation ou la réactivation d'un gène par mutation ou l'amélioration du taux d'expression d'un gène. Il est possible, selon l'invention, de remplacer une phase codante ou une partie d'une phase codante par une séquence hétérologue qui commence au codon d'initiation du gène remplacé afin que l'expression du gène inséré remplace entièrement l'expression du gène remplacé. Ceci évite la formation de protéines de fusion qui pourrait être indésirable chez un animal transgénique non humain.

Selon ce mode de réalisation de l'invention, l'ADN d'insertion peut comporter entre les séquences flanquantes une séquence codante hétérologue dépourvue de promoteur, la séquence codante étant autre qu'un gène codant pour un agent de sélection. L'ADN d'insertion peut comporter en outre, en aval de la séquence codante et toujours entre les séquences flanquantes, un gène codant pour un agent de sélection, associé à un promoteur permettant son expression dans la cellule cible.

De cette manière, la séquence codante hétérologue peut être insérée derrière un promoteur endogène qui a les propriétés souhaitées, par exemple une certaine spécificité d'expression, ou grille de transcription etc, la sélectabilité des cellules transformées étant entièrement indépendante de l'expression de la séquence codante hétérologue. Ce type de construction permet, par exemple, de sélectionner les transformants même si le gène remplacé par la séquence codante hétérologue n'est pas normalement exprimé dans les cellules cibles. Ceci est particulièrement important dans la production d'animaux transgéniques non humains à partir de cellule E.S. non humaines ("Embryonic Stem Cells") puisqu'une proportion importante des gènes reste inactive jusqu'à un stade plus avancé du développement de l'animal non humain. Le gène HOXC8 (également connu sous le nom Hox-3.1) est un exemple de ce type de gène. D'autre part, si la séquence codante code pour une protéine facilement décelable, par exemple, la β-Gal, le développement de la grille de transcription du gène endogène remplacé peut être suivi. Le vecteur pGN est un exemple de ce type de construction.

Selon un autre mode de réalisation de l'invention, l'ADN d'insertion peut comporter une séquence régulatrice étrangère. Le site d'insertion et, par conséquence, les séquences flanquantes sont choisies en fonction du but désiré, à savoir soit l'insertion de la séquence régulatrice étrangère pour donner un effet de "double promoteur" avec la séquence régulatrice endogène, ou soit le remplacement d'un promoteur endogène par le promoteur étranger. La séquence codante qui se trouve sous le contrôle de la séquence régulatrice peut être endogène.

Une autre possibilité serait l'insertion ciblée d'un ADN étranger qui comporte à la fois une séquence régulatrice et une séquence codante. Il est possible que la séquence régulatrice soit celle qui est naturellement associée avec la séquence codante.

Le procédé de l'invention met en oeuvre un vecteur contenant deux séquences "flanquantes" de part et d'autre du gène étranger. Ces séquences flanquantes ont au moins 150 paires de bases et sont de préférence inférieures à la longueur du gène receveur. Il est essentiel que ces deux séquences flanquantes soient homologues aux deux séquences génomiques qui jouxtent le site d'insertion souhaité. La séquence flanquante du vecteur qui se trouve en amont du gène étranger à introduire, est normalement homologue à la séquence génomique qui est située du côté 5' du site d'insertion. De la même manière, la séquence flanquante du vecteur qui se trouve en aval du gène étranger, est normalement homologue à la séquence génomique qui est située au côté 3' du site d'insertion.

Il est possible d'introduire des séquences "intercalantes" entre l'une ou l'autre des séquences flanquantes et le gène étranger, par exemple des séquences permettant la sélection des transformants, des marqueurs, des séquences permettant le clonage du vecteur, etc...

La position de ces séquences intercalantes vis-à-vis du gène étranger doit pourtant être choisie afin de ne pas empêcher l'expression du gène étranger, en particulier de la séquence d'ADN codante étrangère sous le contrôle du promoteur endogène ou, à l'inverse, la séquence codante d'ADN endogène sous le contrôle d'éléments de régulation étrangers apportés par la séquence d'insertion.

Malgré la présence des séquences flanquantes, qui encourage une recombinaison homologue, il est possible qu'un certain nombre d'intégrations se fasse au hasard. Afin de vérifier que l'insertion ciblée a bien eu lieu dans le site ciblé et non pas dans un autre endroit, la technique du "Polymerase Chain Reaction" (P.C.R.) (voir Ref. 10) est utilisée pour amplifier la séquence d'ADN du locus où l'insertion se fait, ou aurait dû se faire. De cette façon, seuls les clones transformés à la suite d'une recombinaison homologue sont sélectionnés.

Les séquences flanquantes du vecteur sont bien évidemment choisies en fonction du site d'insertion désiré pour que la recombinaison homologue puisse avoir lieu. Le cas échéant, les séquences flanquantes peuvent comporter des séquences répliques du promoteur endogène et/ou des modifications aux séquences qui précèdent le codon d'initiation pour améliorer le taux de traduction (séquences amont) et séquences répliques des séquences de terminaison notamment des sites de polyadénylation (séquences aval).

Le gène d'insertion peut être n'importe quel gène d'intérêt. On citera comme exemples non-limitatifs, le gène lacZ (comme dans le modèle décrit plus loin), les gènes codant pour l'interleukine ou l'interféron, les gènes de récepteur, par exemple de l'acide rétinoique ou beta-3 adrénergique ou de H.I.V., et des gènes connus comme étant liés à certaines maladies, par exemple la myopathie, etc...

Les cellules eucaryotes qui ne sont ni des cellules germinales humaines ni des cellules embryonnaires humaines transformées par le procédé de l'invention sont aussi englobées par la présente demande.

Selon une variante préférée de l'invention, les cellules eucaryotes sont des cellules souches embryonnaires non humaines (voir Ref. 14 et 15).

En effet, une cellule E. S. non humaine mutée peut être injectée dans un embryon non humain précoce qui, après réimplantation, pourra naître sous une forme chimère. Si la lignée germinale est colonisée par la cellule non humaine mutée, l'animal non humain chimère transmettra la mutation à sa descendance. Par la suite, on pourra observer les effets de cette mutation, à l'état homozygote chez certains individus, non humains sur leur développement, leur comportement, leur métabolisme, leur pathologie, etc..

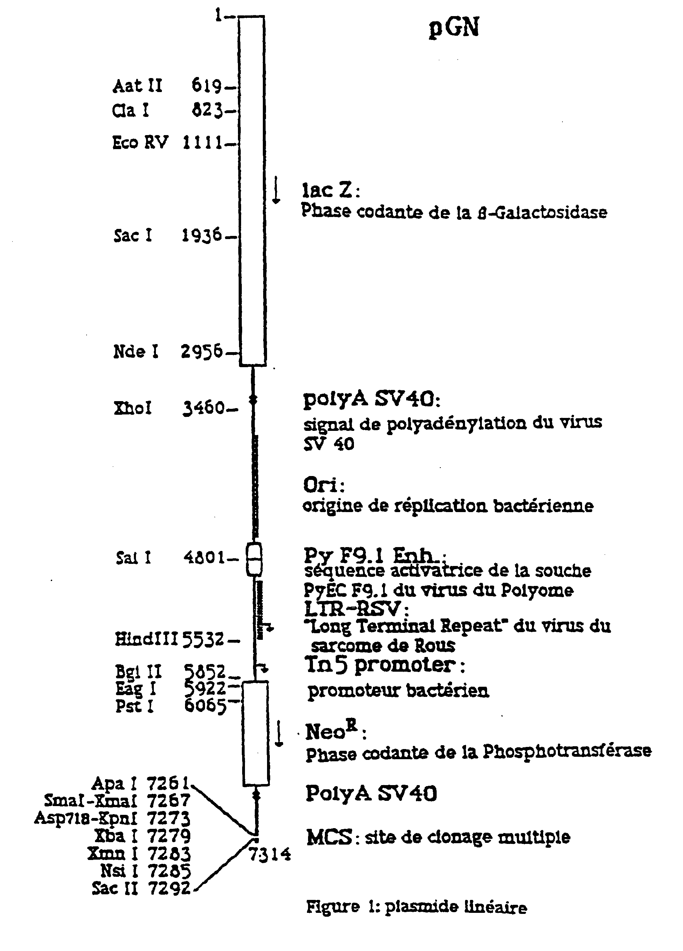

La figure 1 montre le plasmide pGN, qui fait partie de l'invention.

Les figures 2 a et b montrent les molécules pGMA et pGMD respectivement construites à partir du plasmide pGN par rapport au gène HOXC8 (Hox-3.1). Ces plasmides sont des plasmides de mutagénèse. Les deux parties de la phase codante du gène HOXC8 (Hox-3.1) sont représentées, sur le chromosome 15, avec la boîte "homéo" en noir. Les séquences correspondantes de HOXC8 (Hox-3.1) ont été clonées dans le plasmide pGN.
(A : signal de polyadénylation; Enh/Pro : enhancer-promoteur).
07 et 08 figurent les deux oligonucléotides utilisés dans la PCR.

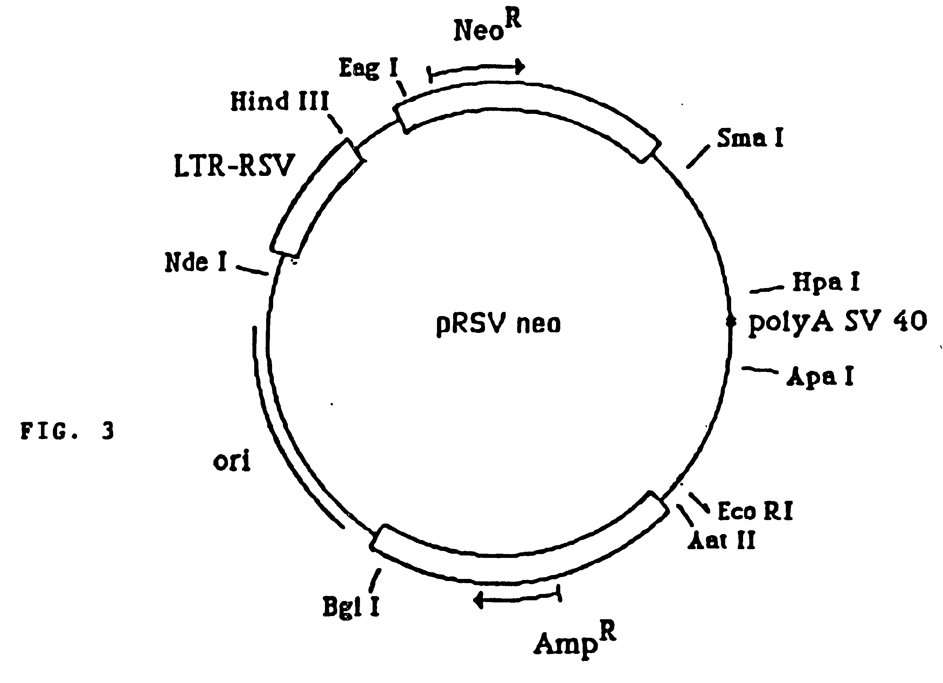

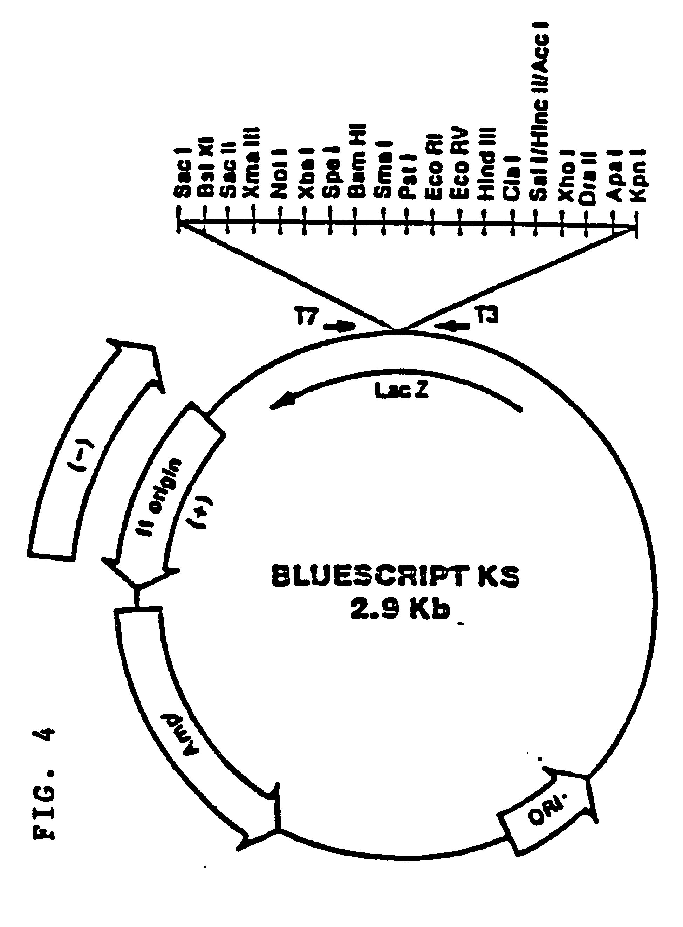

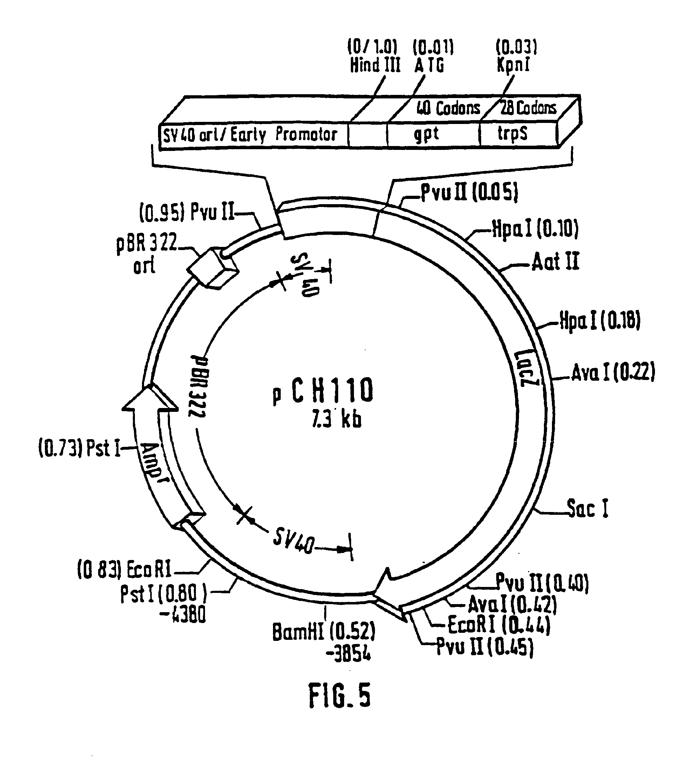

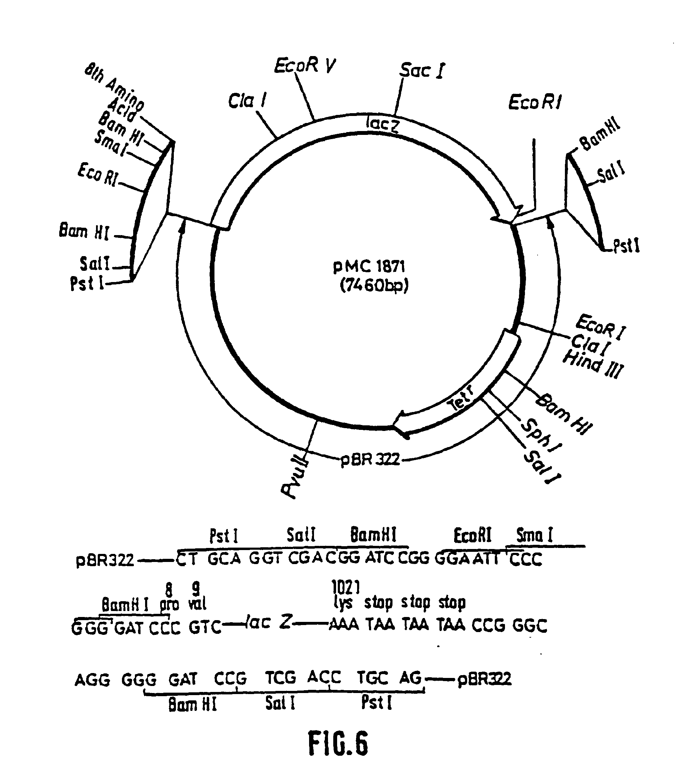

Les figures 3 à 6 montrent les plasmides utilisés dans la construction du pGN.

La figure 7 illustre la détection de recombinaison homologue avec la technique Réaction de Polymérase en Chaîne (P.C.R.) sur des cellules E.S. transfectées.

La figure 8 (a) et (b) montre des analyses de Southern de clones individus positifs (L5 et F2) et cellules E.S. (C.C.E.).

Le procédé de l'invention est d'application industrielle très large et peut varier selon la nature du gène étranger introduit, par exemple pour la production d'animaux transgéniques non humains.

La génétique des mammifères va progresser de manière considérable grâce à la possibilité récente de mutagéniser spécifiquement n'importe quel gène, permettant ainsi de mieux définir son rôle. Par cette technologie qui fait intervenir recombinaison homologue et cellules E.S. non humaines, des informations précieuses seront apportées sur des oncogènes, des facteurs de croissance, des facteurs de transcription, etc..., gènes qui concernent des sujets très actuels de la recherche fondamentale ou la recherche appliquée. Un débouché important pour la recherche médicale est la possibilité de reproduire une maladie humaine dont la détermination génétique est connue (certaines maladies humaines à pathologie, telle la myopathie de Duchesne) ceci afin de mieux en étudier les mécanismes et de rechercher une thérapeutique.

En appliquant le procédé de l'invention, un gène connu comme étant responsable d'une certaine maladie est inséré d'une manière ciblée dans le génome d'une cellule E.S. non humain. L'animal transgénique non humain qui est produit à la suite présente un modèle utile de cette maladie.

Si nécessaire, et comme décrit plus haut, les fonctionnements génétiques normaux peuvent être sensiblement maintenus, malgré l'insertion du gène étranger.

Une autre application du procédé de l'invention consiste en le marquage génétique d'animaux, non humains. Cette application consiste à insérer un gène d'insertion qui est facilement décélé e.g. le gène lacZ et qui peut donc jouer le rôle d'un marqueur cellulaire. De cette manière, des études de filiation e.g. chez des animaux non humains de concours sont facilitées, et la race peut être suivie.

L'insertion du gène lacZ comme gène d'insertion rend aussi possible des études de promoteur. Grâce à la possibilité de déceler l'activité β-galactosidase, l'activité et la spécificité de différents promoteurs endogènes peuvent être étudiées en ciblant différents sites dans le même type ou différents types de cellules. Les mêmes études pourront être effectuées sur un organisme entier, au cours du développement ou à l'état adulte, en utilisant les techniques d'animaux non humains, chimères ou transgéniques.

Les inventeurs ont constaté d'une manière surprenante que la fréquence de recombinaison homologue n'est pas affectée par l'insertion de fragments de taille importante, par exemple le Lac Z. Cette observation a suggéré aux inventeurs que la technique de recombinaison homologue serait bien adaptée à l'insertion d'autres gènes hétérologues qui sont de taille importante.

L'invention vise également l'application du procédé de remplacement ou d'insertion spécifique pour la thérapie génique.

Grâce à la possibilité de modifier le génome d'un animal non humain, le procédé de l'invention peut donc également être utilisé en tant que "thérapie génique". Les utilisations les plus évidentes consisteraient à inactiver les gènes de récepteurs d'agents infectieux (virus ou bactéries) ou toxiques. Si cette mutagénèse s'avérait léthale, il faudrait rétablir la fonction perdue sans rétablir la sensibilité aux agents nuisibles. Un gène modifié codant pour un tel récepteur pourrait être réintroduit dans la cellule mutée, à moins que la modification puisse être provoquée par la recombinaison homologue. Cette modification du patrimoine génétique conférerait à l'animal non humain une immunité contre la maladie considérée.

Ce protocole peut aussi intervenir dans le cadre d'auto-greffe. Des cellules, malades ou saines, et qui ne sont ni des cellules germinales humaines, ni des cellules embryonnaires humaines prélevées sur un patient, pourraient être soignées et immunisées, puis réimplantées chez le même individu.

La technique de l'invention se prête aussi aux études d'activité de produits pharmaceutiques présumés avoir une activité à l'égard des produits d'expression d'un gène pathologique lié à une maladie. Dans ce cas, le gène d'insertion est constitué par le gène pathologique ou un fragment de celui-ci et on administre à l'animal transgénique non humain le produit pharmaceutique à tester, en vue d'évaluer son activité sur la maladie.

L'invention va être illustrée en faisant référence au plasmide pGN et son utilisation dans l'insertion ciblée d'un gène étranger (lacZ, codant pour l'enzyme β-glactosidase d'E.Coli) dans le génome d'une cellule d'E.S. de souris. Le gène lacZ a été choisi en raison du fait que son expression peut être facilement décelée et est simplement à titre illustratif.

La phase codante de l'enzyme β-galactosidase d'E.Coli (lacZ; 1-3057), fusionnée avec une séquence génomique (7292-3) du gène murin HOXC8 (Hox.3-1) (Ref. 1), débute par le codon d'initiation de ce gène. En effet, la séquence qui précède le codon d'initiation de HOXC8 (Hox-3.1) est identique à la séquence consensus observée chez les vertébrés (Ref. 2), permettant ainsi un meilleur taux de traduction de la β-galactosidase dans les cellules de vertébrés. Le gène lacZ est suivi d'un signal de polyadénylation par exemple du virus SV 40, comme la plupart des gènes eucaryotes, afin de stabiliser les ARNₛ messagers.

L'activité de la β-galactosidase d'E.Coli, qui est fonctionnelle dans les cellules eucaryotes, peut être décelée de différentes manières. Des cellules exprimant le gène lacZ prennent une coloration bleue, après fixation, en présence de X-Gal, qui est un substrat de la β-galactosidase (Ref. 3). Un nouveau substrat, le FDG (flurorosceine di-β-galactopyranoside), permet de déceler et de doser l'activité β-gal. tout en gardant les cellules vivantes (Ref. 4). Les cellules exprimant lacZ accumulent un produit fluorescent et peuvent être isolées à l'aide d'un trieur de cellules ou FACS (fluorescence-activated cell sorter).

L'unité de transcription du gène de résistance à la néomycine provient, en grande partie, du plasmide pRSV neo (Ref. 5). Le LTR (long terminal repeat) du virus du sarcome de Rous procure des séquences activatrice et promotrice très puissantes dans de nombreuses cellules eucaryotes (Ref. 6). Du transposon bactérien Tn5, viennent un promoteur actif dans E.Coli et la phase codante de l'enzyme phosphotransférase (Ref. 7), qui est suivie du signal de polyadénylation du virus SV40. Le même gène sous le double contrôle des promoteurs RSV et Tn5 peut conférer la résistance à la néomycine ou la kanamycine aux bactéries et la résistance au G418 aux cellules eucaryotes.

Par l'effet d'une simple mutation ponctuelle, l'unité B des séquences activatrices (enhancer) de la souche PyEC F9.1 du virus du Polyome est devenue beaucoup plus active dans différents types de cellules, et en particulier dans les cellules de carcinome embryonnaire (EC) (Ref. 8). Deux copies de cet enhancer Py F9.1 ont été insérées en tandem dans le plasmide pGN, en amont du LTR-RSV, et dans l'orientation "promoteur tardif" de la région régulatrice du Polyome.

Afin d'améliorer le taux de traduction de la phosphotransférase, la séquence précédent le codon d'initiation a été modifiée lors d'une mutagénèse par oligonucleotide. Ainsi la séquence T T C G C A U G est devenue G C A C C A U G, correspondant beaucoup mieux à la séquence consensus d'initiation de la traduction chez les vertébrés (Ref. 2).

Les améliorations apportées à l'unité de transcription du gène de résistance à la néomycine ont pu être estimées en transfectant des cellules souches embryonnaires (ES) de souris. A molarité égale en plasmide, une construction avec les enhancer Py F9.1 a produit 7,5x plus de clones résistants au G418 que le pRSV neo et 2 à 3x plus que le pMC1 Neo décrit par Capecchi et al (réf. 13). A nouveau, le nombre de clones a été augmenté 60x, soit 450x par rapport au pRSV neo, en modifiant la séquence d'initiation de la traduction. La recombinaison homologue peut être un événement assez rare, selon les conditions expérimentales appliquées (p. ex 1/1000 pour HPRT, réf. 13). Un vecteur présentant une efficacité de sélection élevée est donc très utile, d'autant plus que les conditions d'électroporation donnent lieu principalement à l'intégration d'une seule copie.

Le plasmide pGN contient, en outre, une origine de réplication bactérienne de type colE1, pBR322, qui permet les clonages et les préparations dans E.Coli.

Enfin, un site de clonage multiple (M.C.S.), synthétisé in vitro, qui ne contient que des sites de coupure uniques dans pGN, a été inséré en amont de lacZ, afin de faciliter les utilisations de ce plasmide.

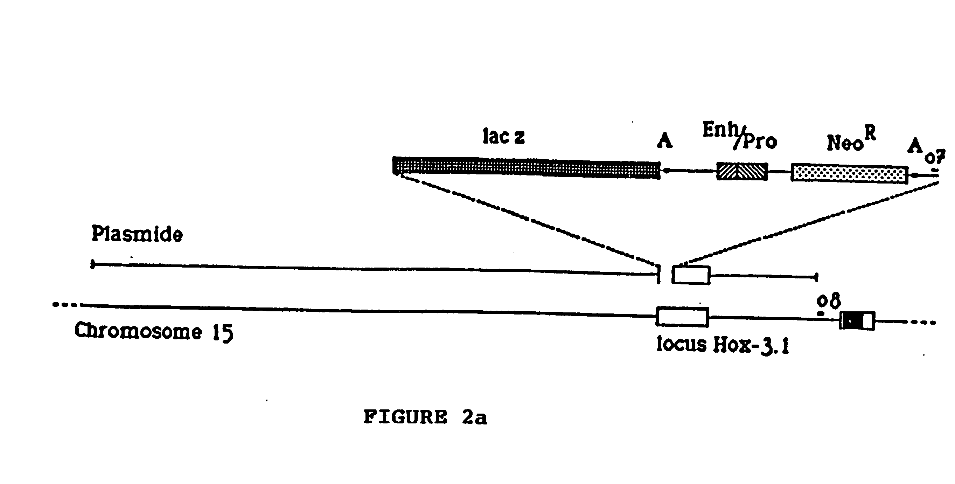

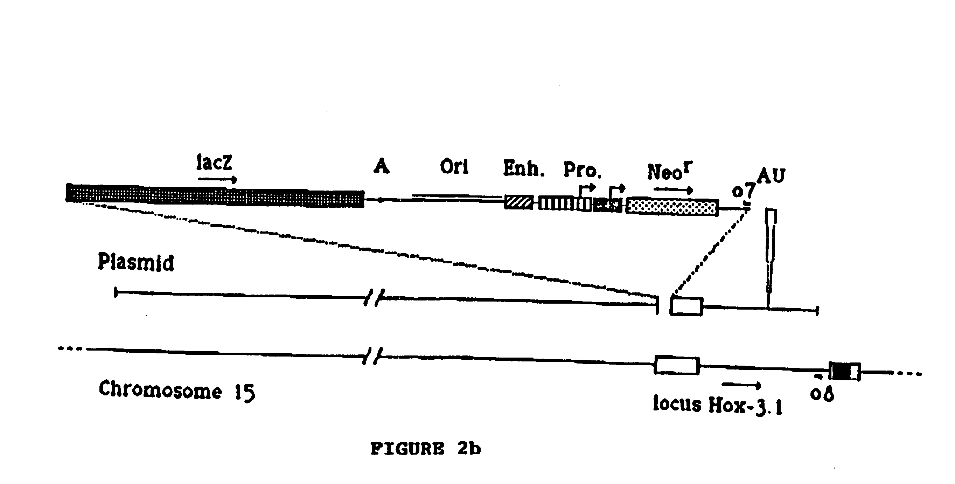

Les séquences "flanquantes" plasmidiques qui provoquent la recombinaison homologue sont ajoutées aux extrémités du plasmide pGN après linéarisation du plasmide en amont de lacZ, par un site du MCS (voir fig. 2). En l'occurence, les séquences flanquantes choisies sont homologues aux séquences chromosomiques issues de HOXC8 (Hox-3.1) devant ultérieurement intervenir dans la recombinaison homologue.

La figure 2 situe la molécule construite à partir du plasmide pGN par rapport au gène HOXC8 (Hox-3.1). Dans ce cas, une recombinaison entre les séquences plasmidiques et chromosomales de HOXC8 (Hox-3.1) resulterait en une insertion au début de la phase codante de ce gène, donc à son inactivation totale.

Le plasmide pGN rassemble plusieurs avantages pour cette méthodologie, qui est applicable à n'importe quel gène. L'évènement de recombinaison homologue pouvant être assez rare (de l'ordre de 1 pour 1000 intégrations non-homologues), il est nécessaire de pouvoir analyser un grand nombre de clones dont la résistance au G418 soit suffisament forte pour s'exprimer dans n'importe quelle partie du génome. Les modifications apportées à l'unité de transcription de la phosphotransférase répondent parfaitement à ces problèmes. La méthode de mutagénèse par recombinaison homologue équivaut à inactiver un gène par une insertion ou une substitution, mais le plasmide pGN présente l'avantage supplémentaire de pouvoir substituer l'expression de la β-galactosidase à celle du gène muté. Enfin, le MCS facilite les clonages de fragments génomiques.

### EXEMPLES :

### I - Construction du plasmide pGN

Les plasmides intermédiaires sont numérotés selon leur étape.

### 1° étape :

### Insertion d'un site Xho I dans le site Bql I de pRSV neo

Insertion d'un linker Xho I dans le site Bgl I de pRSV neo, rempli par le fragment Klenow de l'ADN polymérase d'E.Coli.

### 2° étape :

### Insertion d'un site Cla I dans le site Nde I du plasmide p1

Insertion d'un linker Cla I dans le site Nde I de pl, rempli par la polymérase Klenow.

### 3° étape :

### Insertion d'enhancer Py F9.1 dans le site Cla I du plasmide p2

Insertion d'enhancer Py F9.1 Pvu II-Pvu II isolé par un site unique, Acc I, dans le site Cla I de p2. Sélection d'un clone contenant deux enhancers orienté dans le sens "promoteur tardif".

### 4° étape :

### Déletion Sma I-Hpa I du plasmide p3

Les deux enzymes, donnant des extrémités "bouts-francs", peuvent être ligués directement. Cette délétion enléve l'intron de l'antigène t de SV 40, qui n'est pas très utile, et diminue de manière appréciable la taille de l'unité de transcription de la phosphotransférase.

### 5° étape:

### Insertion d'un site Xho I dans le site Bam HI de pCH110

Insertion d'un linker Xho I dans le site Bam HI du plasmide pCH 110 (Pharmacia), rempli par la polymlérase Klenow.

### 6° étape:

### Insertion du 3' lacZ-polyA SV 40 dans le plasmide p4

La partie 3' de la phase codante de la β-galactosidase, suivie du signal de polyadénylation du virus SV 40 est isolée du plasmide p5 par les sites Xho I-Aat II et clonée dans le plasmide p4 par les mêmes sites.

### 7° étape :

### Insertion du 5' lacZ dans le vecteur KS-

La partie 5' de la phase codante de la p-galactosidase est isolée du plasmide pMC 1871 (Pharmacia) par les sites Pst I-Sac I et clonée dans le vecteur KS- (Stratagene) par les mêmes sites.

### 8° étape:

### Fusion d'une séquence génomique HOXC8 (Hox-3.1) avec le 5' lacZ

Une séquence génomique du gène HOXC8 (Hox-3.1), clonée dans le vecteur KS-, est purifiée par digestions successives par l'enzyme Sac I, puis par la nucléase Mung bean et enfin par l'enzyme Apa I. Cet insert est fusionné avec la partie 5' de la phase codante de la β-galactosidase par clonage dans le plasmide p7 digéré par Apa I-Sma I. La protéine ainsi fusionnée contient le codon d'initiation de la traduction du gène HOXC8 (Hox-3.1) suivi de la phase codante de la β-galactosidase (vérifiée ensuite par séquençage).

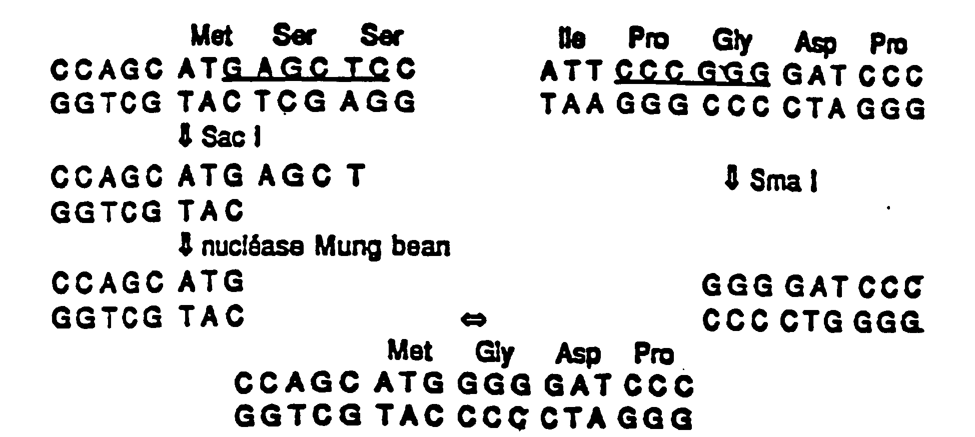

### 9° étape :

### Insertion de HOXC8 (Hox-3.1)-5' lacZ dans le plasmide p6

La fusion HOXC8 (Hox-3.1)-5' lacZ est isolée du plasmide p8 par les sites Apa I-Sac I et clonée dans le plasmide p6 par les mêmes sites. Ce clonage a pour effet de reconstituer la phase codante de la β-galactosidase dans sa totalité.

### 10° étape :

### Insertion du gène Neo^{R} dans le vecteur KS+

Le gène de résistance à la néomycine (promoteur bactérien et phase codante de la phosphotransférase) est isolée du pRSV neo par les sites Hind III-Eco RI et clonée dans le vecteur KS+ (Stratagene).

### 11° étape :

### Mutagénèse de la séquence d'initiation de Neo^{R} dans p10

La séquence d'initiation de la traduction de la phnosphotransférase est modifiée pour être identique à la séquence consensus observée chez les vertébrés et permettre ainsi un taux supérieur d'initiation de la traduction donc une résistance accrue au G418 pour les cellules de mammifères. La modification crée également un site Apa LI qui permet de contrôler l'efficacité de la mutagénèse.

Apa LI
GTTTCGCATG ⇒ GTGCACCATG

Un oligonucléotide (CTTGTTCAATCATGGTGCACGATCCTCA) comportant une région de misappariement avec la séquence du pRSV neo (soulignée) est synthétisé (Gene Assembler, Pharmacia) puis phosphorylée par la polynucléotide kinase du bactériophage T4. Une matrice simple brin du plasmide p10 est préparée grâce à l'origine fl du plasmide KS+ et hybridée avec l'oligonucléotide de mutagénèse. Le deuxième brin est synthétisé et réparé par la polymérase Klenow et l'ADN ligase du bactériophage T4. Aprés transformation de bactéries, les clones mutés sont criblés à l'aide de l'oligonucléotide marqué au ³²P. La mutagénèse a été vérifiée en digérant par Apa LI ainsi que par séquençage.

### 12° étape :

### Remplacement de la séquence d'initiation dans le plasmide p9

Un fragment contenant la séquence modifiée d'initiation de la traduction du gène de résistance à la néomycine est isolée du plasmide p11 par les enzymes Hind III-Eag I et clonée dans le plasmide p9 par les mêmes sites.

### 13° étape :

### Insertion du site de clonage multiple dans le plasmide p12

Deux oligonucléotides complémentaires sont synthétisés (Gene Assembler, Pharmacia) puis phosphorylés. Après appariement, le MCS est cloné dans les sites Apa I- Sac II du plasmide p12 grâce à ses extrémités cohésives

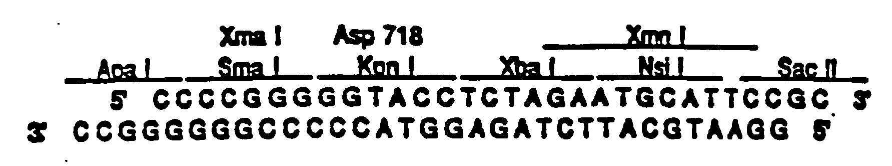

Le site de clonage multiple a également été vérifié par séquençage.

### II - Addition des séquences "flanquantes" aux extrémités du plasmide pGN linéarisé en amont de lacZ' par un site du M.C.S

Les séquences flanquantes utilisées ont été choisies en fonction du site d'insertion souhaité (par exemple, HOXC8 (Hox-3.1), voir Fig. 2 a et b pGMA et pGMD).

Dans la construction du plasmide de mutagénèse pGMD, deux bras d'ADN homologue au locus HOXC8 (Hox-3.1) ont été clonés aux sites Apa I-Nsi I et Nsi I-Sac II du vecteur pGN. Le bras 5' commence au site Sac II (CCGCGG)au nucléotide 219 de l'ADNc c21 de HOXC8 (Hox-3.1). Ce fragment s'étend sur 6.8 kb en 5' jusqu'au premier site BamHI. Le bras 3' commence au site Apa 1 (GGGCCC) au nucléotide 885 de l'ADNc c21. Ce fragment s'étend sur 1.5 kb en 3' jusqu'au premier site PstI. Un linker NsiI a été inséré dans le site BamHI du fragment 5' et dans le site PstI du fragment 3'. Les bras 5' et 3' ont été clonés dans le vecteur pGN dans les sites Nsi I-Sac II et Apa I-Nsi I, respectivement. La séquence de l'ADNc de HOXC8 (Hox-3.1) c21 est publiée (réf. 1).

Le plasmide de mutagénèse est linéarisé par digestion avec Nsi I avant électroporation de cellules E.S. Ses extrémités sont formées des deux bras génomiques clones aux sites Apa I-Nsi I et Nsi I-Sac II du vecteur pGN.

Le plasmide pGMD ne présente pas de signal de polyadénylation après le gène de résistance mais, en revanche, présente une séquence riche en AU responsable d'une dégradation sélective de mRNA, insérée dans la séquence de l'intron du HOXC8 (Hox-3.1) du plasmide.

Un autre plasmide de mutagénèse, pGMA, présente la même structure que le pGMD mais contient les signaux de polyadénylation et de terminaison de transcription du SV40 et ne présente pas la séquence AU de dégradation de mRNA en aval du gène Neo^{r}. Ces modifications avaient pour but de réduire le taux de transcrits de Neo^{r} dans des clones issus d'intégration au hasard. En revanche, des clones issus d'événements de recombinaison homologue entre pGMD et un locus HOXC8 (Hox-3.1) devrait avoir une croissance inaltérée pendant la sélection au G418, la séquence AT de dégradation de mRNA étant éliminée par le procédé de recombinaison même, ou epissée avec l'intron HOXC8 (Hox-3.1).

Dans les étapes expérimentales qui suivent, le protocole décrit par Thompson et al. 1989, a été suivi pour la production d'animaux chimères.

### III - Transfection de cellules embryonnaires de souris

La méthode décrite par Thompson et al. 1989, a été utilisée pour transfecter des cellules embryonnaires de souris. L'utilisation de la technique de l'électroporation assure l'introduction d'une seule copie du gène étranger (lacZ) par cellule. Après transfection, plusieurs clones exprimant la β-galactosidase ont été isolés.

Les plasmides de mutagénèse pGMD et pGMA ont été linéarisés et introduits par électroporation dans des cellules E.S. afin de favoriser l'insertion d'une copie seulement dans le génome (réf. 11).

Les transfections initiales ont été effectuées pour comparer l'efficacité de ciblage du HOXC8 (Hox-3.1) des plasmides pGMA et pGMD (voir tableau I).

**Tableau I**

| Recombinaison homologue dans le gène HOXC8 | | | | |
|---|---|---|---|---|
| (Hox-3.1) | | | | |
| Exp. | Plasmide de mutagénèse | N° de l'ensemble analysé | Nb de clones formant l'ensemble | Nb de résultats P.C.R. positifs |
| I | pGMA | 3 | 600 | 0(2) |
| II | pGMD | 5 | 250 | 3(5) |
| III | pGMD | 84 | 2-3 | 5(5) |

La lignée cellulaire E.S. "C.C.E." (réf. 16) a été maintenue d'une manière continue sur des couches nourricières fibroblastiques (réf. 17). Pour les expériences I et II, 1.5 x 10⁷ cellules E.S. dans 1.5 ml HeBS ont été électroporées (réf. 11) à 200 V, avec 40 mg de plasmide linéarisé, puis étalées sur quatre boîtes de cultures (diamètre 100 mm). Pour l'expérience III, le choc a été effectué dans les mêmes conditions mais un quart des cellules ont été étalées sur quatre plaques à 24 puits. Le lendemain, 250 *µ*g ml⁻¹ G418 ont été ajoutés. Chaque transfection a donné lieu à environ 2400 clones avec pGMA et environ 1000 clones avec pGMD.

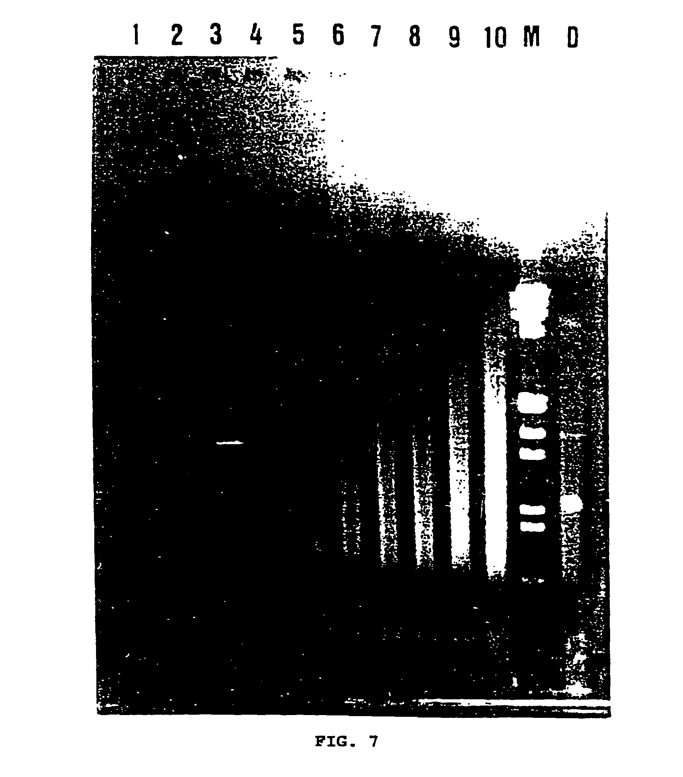

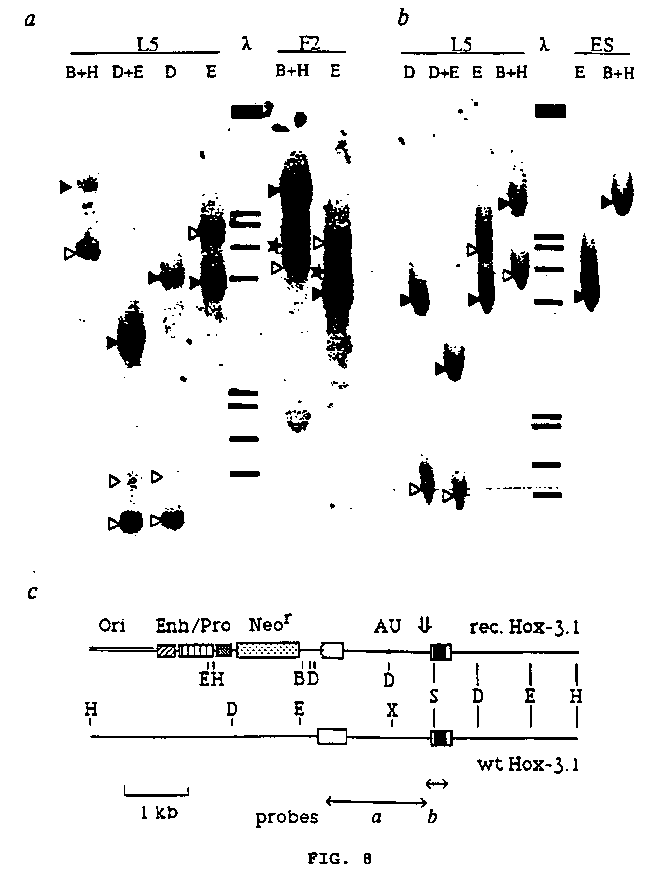

Le nombre moyen de clones de cellules E.S. résistantes au G418 dans chaque ensemble est indiqué dans le tableau I, ainsi que le nombre d'ensembles donnant un résultat positif avec la technique P.C.R. Un résultat positif signifie q'une bande de 1.6 Kb a pu être observée sur un gel d'agarose coloré de bromure d'ethidium (voir fig. 7). Le nombre d'ensembles donnant un signal positif après une analyse de Southern du mélange P.C.R. et hybridation d'une sonde spécifique qui ne contenait pas les séquences des amorces est indiqué entre parenthèses (fig. 8).

### Détection de la recombinaison homologue avec la P.C.R.

P.C.R. a été effectuée sur 10⁵ cellules d'un ensemble de 250 clones de la transfection II (voir voie D de la fig. 7). Dans les autres voies, quatre ensembles de la transfection III ont été analysés ensemble en mélangeant environ 4 x 5000 cellules. Les amorces 07 et 08 utilisées dans la P.C.R. entourent la séquence 3'HOXC8 (Hox-3.1) du plasmide de mutagénèse (fig. 2). Le fragment de 1.6 Kb recouvrant cette séquence 3' ne peut être amplifié que dans le cas d'une recombinaison homologue. Les voies 2, 3 et D illustrent des résultats positifs.

L'ADN de clones E.S. a été préparé au moment de la réplique sur filtre en utilisant la méthode "boiling-proteinase K digestion boiling" (réf. 18). 40 cycles d'amplification (40 secondes à 94°C, 1 minute à 60°C, 7 minutes à 72°C) ont été effectués dans un mélange réactionnel de 100 µl, contenant 67 mM Tris-HCL (pH 8.6), 16.7 mM (NH₄)₂SO₄, 6.7 mM MgCl₂, 10 mM 2-mercaptoethanol, 0.01 % (p/v) gélatine, 200 µM dATP, dTTP et dCTP, 100 µM dGTP, 100 µM 7-deaza dGT, 600 ng de chaque amorce (07 : AACTTCCCTCTCTGCTATTC et 08 : CAGCAGAAACATACAAGCTG) et 3U polymérase Taq (Perkin Elmer Cetus), couvert de 100 µl paraffin. La moitié du mélange de réaction a été appliquée sur un gel d'agarose 0.7 % coloré au bromure d'ethidium. Le marqueur de taille est un digest Eco RI + Hind III d'ADN lambda.

### Analyses de Southern

Trois clones indépendants de cellules E.S. contenant le HOXC8 (Hox-3.1) muté (identifié par P.C.R.) ont été isolés des ensembles positifs en utilisant des pipettes. Leur ADN a été examiné par analyse de Southern après digestion avec les enzymes de restriction indiqués dans la figure 8, afin de confirmer le ciblage spécifique et de faire la distinction entre les loci recombinés et sauvages. Deux sondes différentes ont été utilisées dans l'analyse de l'extrémité 3' des loci HOXC8 (Hox-3.1) dans les clones mutés et dans les cellules E.S. non-mutées agissant comme témoin (fig. 8 c). La première sonde "a" était contenue dans les séquences HOXC8 (Hox-3.1) du plasmide de mutagénèse et démontrait le nombre d'intégrations de vecteur et leurs liaisons physiques. Un des trois clones recombinés contenait en outre une copie du plasmide intégrée au hasard (fig. 8 a, clone F2). La deuxième sonde "b" qui n'était pas contenue dans le vecteur de mutagénèse faisait la distinction entre les allèles HOXC8 (Hox-3.1) recombinées et sauvages (fig. 8 b). Le locus recombiné HOXC8 (Hox-3.1) présentait, avec les deux sondes, l'image d'hybridation attendue à partir des cartes de restriction du vecteur de mutagénèse et du locus intact. En outre, l'existence de deux domaines de recombinaison dans le bras 3' du vecteur a été confirmée par la présence ou l'absence de la séquence AT dans le locus HOXC8 (Hox-3.1) recombiné (par exemple fig. 8, clone L5). L'extrémité 5' du locus HOXC8 (Hox-3.1) a également été analysée pour l'événement de recombinaison homologue. Des enzymes de restriction ne présentant pas de sites dans la séquence HOXC8 (Hox-3.1) 5' de 6.8 Kb du vecteur de mutagénèse ont été utlisés dans la digestion des ADNs des clones recombinés. Ces ADNs ont ensuite été soumis à une électrophorèse dans un champ pulsé pour différencier les fragments de poids moléculaire élevé. Une analyse de Southern de ce gel a également indiqué les allèles recombinées correctement et les allèles HOXC8 (Hox-3.1) sauvages, en utilisant une sonde présentant une séquence en amont du plasmide de mutagénèse.

Les analyses de Southern ont démontré q'une allèle du gène HOXC8 (Hox-3.1) a été recombinée comme prévu. La recombinaison homologue était équivalente à un double "crossing-over" entre les bras génomiques du plasmide de mutagénèse et les séquences chromosomales homologues (Fig. 2).

Dans les clones recombinants, le gène lac Z a été placé sous le contrôle des séquences promotrices et régulatrices du HOXC8 (Hox-3.1) en amont du codon AUG, mais les signaux 3' de maturation de mRNA provenaient du SV40. Dans ces clones recombinés, l'expression de lac Z n'était pas décelable par coloration avec β-Gal, ce qui est cohérent avec l'absence de transcription de HOXC8 (Hox-3.1) dans des cellules E.S. déterminée par analyse de protection de RNase. L'activité de β-Gal pouvait être induite dans certaines cellules après 3 ou 4 jours de culture en présence de 5.10⁻⁷M acide rétinoïque, conditions connues comme induisant la transcription de HOXC8 (Hox-3.1) (réf. 19).

En utilisant le vecteur de mutagénèse pGMA, qui présente une homologie totale de 8.3 Kb ADN avec le locus HOXC8 (Hox-3.1), un fragment de 120 pb a été remplacé par une insertion de 7.2 Kb. La fréquence de ce remplacement ciblé (1/900) est comparable à celle obtenue récemment (1/1000) avec HPRT (réf. 13) ou avec En-2 (1/260) (réf. 20), le fragment hétérologue inséré étant cependant dans ces derniers cas d'une taille beaucoup moins importante (1.1 et 1.5 Kb respectivement). D'une manière surprenante, il a été constaté qu'une fréquence de recombinaison homologue très élevée (1/40) a pu être obtenue avec le vecteur pGMD. L'élimination des signaux 3' de maturation de mRNA et l'addition de la séquence de dégradation de mRNA au gène de résistance à la néomycine a eu pour effet de réduire le nombre total de clones résistants au G418 par 2.4 (tableau I). Le rapport de ciblage spécifique était presque 10 fois plus élevé (900/40). Le mécanisme de recombinaison homologue même a dû être affecté dans les expériences avec pGMD. Une explication possible de ces résultats serait qu'une séquence AT de 51 pb pourrait fournir, in vivo, une boucle ouverte dans le plasmide de mutagénèse à cause de sa température de fusion plus basse. Si les séquences HOXC8 (Hox-3.1) voisines du pGMD peuvent être influencées par cette ouverture, de chaque côté de la région AT, elles pourraient réagir d'une manière plus efficace, à l'état simple-brin, avec le locus chromosomal HOXC8 (Hox-3.1). Le modèle de recombinaison mitotique chez la levure suggère qu'il serait initié par un tel échange de brins, bien que le mécanisme de recombinaison homologue reste inconnu chez les eukaryotes plus complexes.

La figure 8 montre les résultats de l'analyse de Southern effectuée sur des clones individus positifs (L5 et F2) et des cellules E.S. (C.C.E.).

Les sondes utilisées n'hybrident qu'avec des séquences HOXC8 (Hox-3.1) incluses dans le vecteur (a) ou exclues du vecteur de mutagénèse (b). L'image d'hybridation du locus HOXC8 (Hox-3.1) recombiné (triangles ouverts) se distingue clairement du locus sauvage (triangles noirs). Les étoiles indiquent les bandes d'hybridation d'une copie du plasmide qui s'est intégrée au hasard. Le marqueur de taille est un digest Eco RI + Hind III d'ADN lambda.

La figure 8(c) montre les cartes de restriction des allèles HOXC8 (Hox-3.1) recombinées (rec.) et sauvages (wt). Les parties du vecteur de mutagénèse et du locus HOXC8 (Hox-3.1) sont indiquées avec les mêmes symboles que ceux utilisés dans la figure 2. Dans ce cas, la séquence AT a été intégrée par recombinaison homologue. La flèche verticale indique l'extrémité 3' du plasmide de mutagénèse. La localisation des sondes "a" et "b" utilisées dans l'analyse de Southern est également indiquée. Les abréviations utilisées dans la figure 8 sont les suivantes : B, Bam HI ; D, Dra I, E, Eco RI ; H, Hind III : S, Sal I ; X, Xho I.

### IV - Production d'embryons chiméres non humains

Une microinjection dans des blastocystes a été effectuée avec deux clones E.S. recombinants contenant un allèle HOXC8 (Hox-3.1) intact et un allèle recombiné, ces clones ne contenaient aucune autre copie du plasmide de mutagénèse. Les kariotypes de ces cellules étaient normaux.

Dix à quinze cellules mutées ont été microinjectées par blastocyste. Après réimplantation dans des mères porteuses, les embryons ont été recueillis à 9.5, 10.5 et 12.5 jours p.c. et analysés pour expression de lac Z. La grille de transcription de HOXC8 (Hox-3.1) à ces stades avait été déterminée au préalable par analyse d'hybridation in situ (réf. 1). Les transcrits HOXC8 (Hox-3.1) sont détectables pour la première fois au stade de gastrulation tardive et sont répartis dans tous les tissus de la partie postérieure de l'animal. Plus tard, la répartition devient progressivement limitée dans l'espace et spécifique au tissu. Au stade de 12.5 jours p.c., la transcription est localisée dans la région cervicale du tube neural, au niveau du coeur. Au cours de l'embryogenèse, la répartition de la transcription de HOXC8 (Hox-3.1) subit donc des modifications. Le stade 10.5 jours p.c. semble être une période de transition, la transcription ayant lieu à la fois dans les deux régions arrières et dans le tube neural cervical.

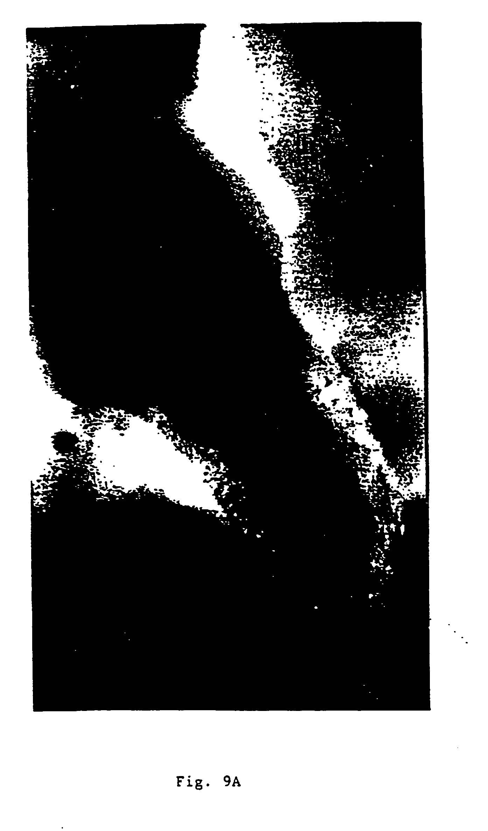

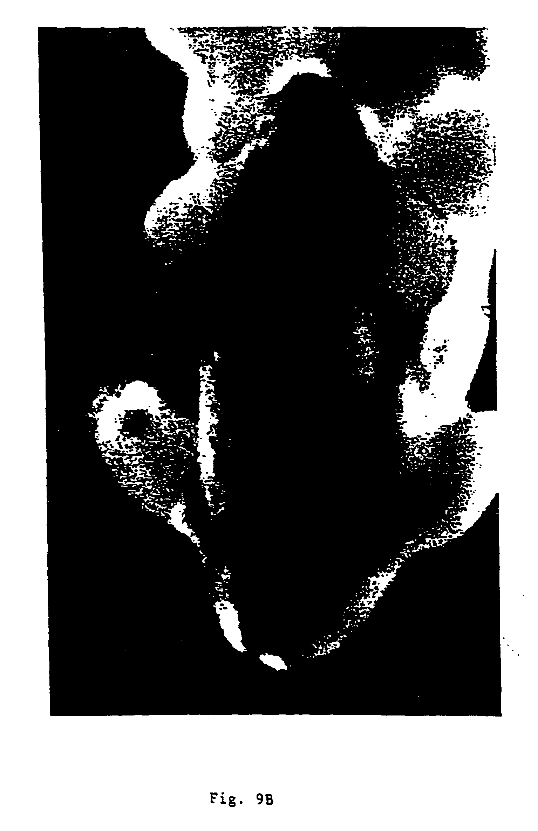

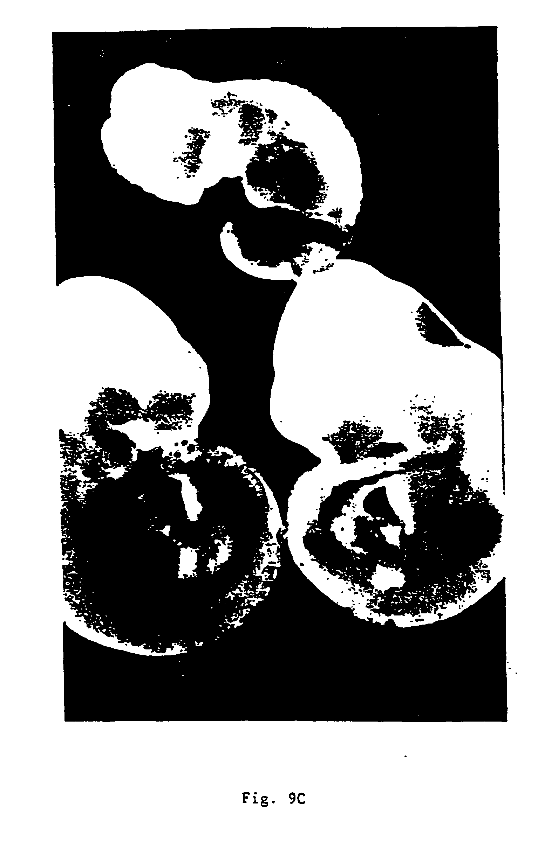

Dans des embryons chimériques de 9.5 et 10.5 jours p.c., la partie caudale au bourgeon postérieur présentait une activité β-Gal intense, tandis que le marqueur n'a jamais été détecté dans la région thoracique antérieure ou la tête (Fig. 9a). Dans la région postérieure, des cellules colorées par le β-Gal ont été observées dans tous les tissus et de toutes les couches embryonnaires. Entre les deux bourgeons qui donnent les membres, des cellules colorées étaient réparties dans des zones restreintes, dans l'ectoderme superficiel (Fig. 9b), comme dans les régions arrières (Fig. 9c) et, en forme de lignes étroites ou rayures, dans le tube neural (Fig. 9b). Ces rayures présentaient une répartition irrégulière et asymétrique sur la paroi du tube neural. La transcription de HOXC8 (Hox-3.1) n'a pas été détectée dans la couche mince de cellules vers la fermeture du tube neural. Ces cellules n'ont peut-être pas résisté aux traitements appliqués lors de l'hybridation in situ. Il a été observé que les cellules de l'ectoderme neural font partie, très tôt, de parties différentes du système nerveux et migrent dans une direction radiale, suivant des mouvements latéraux étroits (réf. 21). Ces résultats sont donc cohérents avec cette observation.

L'expression de Lac Z a donc illustré correctement la première partie de la transcription de l'homéogène HOXC8 (Hox-3.1), c'est-à-dire dans tous les tissus des régions caudales des embryons de 9.5 et 10.5 jours p.c., et a fourni de nouvelles informations concernant le mode de transcription de HOXC8 (Hox-3.1).

En revanche, l'expression de Lac Z n'a pas été observée dans les régions cervicales du tube neural d'embryons chimères de 12.5 jours, ni dans la région antérieure d'embryons de 10.5 jours ; ceci n'était pas le résultat attendu à partir des études d'hybridation in situ. La phase ultérieure de transcription de HOXC8 (Hox-3.1) observée à partir du jour 10.5 dans les zones très localisées du tube neural n'était pas mise en évidence par l'activité de β-Gal. Une explication possible pour ce résultat serait que, bien que l'expression de Lac Z soit sous le contrôle du promoteur HOXC8 (Hox-3.1), les séquences 3' du HOXC8 (Hox-3.1) sont absentes dans le gène reporteur. Il est possible que des séquences 3' du codon d'initiation AUG du HOXC8 (Hox-3.1) aient une influence sur l'expression tardive de HOXC8 (Hox-3.1) dans le domaine antérieur. Un effet de "dosage de gène" pourrait aussi expliquer ce résultat. L'autoactivation de plusieurs homéogènes chez Drosophila a été démontrée génétiquement ou suggérée par la formation de complexes entre l'ADN et les protéines des homeobox.

Si le composant tardif de la transcription de HOXC8 (Hox-3.1) dans le tube neural est maintenu par un mécanisme semblable, l'inactivation d'un allèle pourrait avoir un effet dominant dans les celules de l'ectoderme neural. Puisqu'un allèle seulement produirait la protéine HOXC8 (Hox-3.1), le signal d'activation serait dilué sur les deux promoteurs. La réduction d'autoinactivation dans les deux loci pourrait ainsi conduire à un arrêt total de l'initiation de transcription. Ceci pourrait expliquer pourquoi aucune expression de Lac Z n'a été détectée dans la région cervicale du tube neural d'embryons de 10.5 et 12.5 jours.

### V - Passage de la modification dans la lignée cellulaire germinale : production d'animaux transgéniques non humains.

Les effets en F₁ et en F₂ de la modification apportée par l'insertion ciblée ont été observés après reproduction des chimères. Le passage de la modification dans la lignée cellulaire germinale a été constaté.

### BIBLIOGRAPHIE

1. Le Mouellic, H., Condamine, H. et Brûlet, P. (1988). Genes & Development. 2, 125-135.
2. Cavenir, D.R. (1987). Nucleic Acids Res. 15, 1353-1361.
3. Sanes, J.R. Rubenstein, J.L.R. et Nicolas, J.F. (1986) EMBO J. 5, 3133-3142.
4. Nolan, G.P., Fiering, S., Nicolas, J.F. et Herzenberg, L.A. (1988) Proc. Natl. Acad. Sci. USA 85, 2603-2607.
5. Gorman, C., Padmanabhan, R. et Howard, B.H. (1983). Science. 221, 661-553.
6. Gormann, C.M., Merlino, G.T. Willingham, M.C. Pastan, I. et Howard, B. H. (1982) Proc. Natl. Acad. Sci. USA 79, 6777-6781.
7. Southern, P.J. et Berg, P. (1982) J. Mol. Appl. Genet 1, 327- 341.
8. Herbomel, P., Bourachot, B. et Yaniv, M. (1984). Cell. 39, 653-662.
9. Robertson, E.J. (1987). Teratocarcinomas and embruonic stem cells: A practical approach. IRL Press, Oxford.
10.Kahn, A. (1988). Médecine/Sciences 4, 515-518.
11.G. Chu, Hayakawa H., Berg. P. (1987) Nucleic Acid Research 15, Nr. 3, 1311-1326.
12.Thompson, S., Clarke, A.R., Pow, A.M., Hooper, M.L., Melton, D.W., (1989) Cell, 56, 313-321.
13.Thomas, K.R.,Capecchi, M.R., (1987) Cell, 51 503-512.
14. Evans, M.J., Kaufmann, M.H. (1981) Nature, 292, 154-155,
15. Robertson, E.J., (1986) Trends in Genetics, 9-13
16. Robertson, E., Bradley, A., Kuehn, M. & Evans, M. Nature 323, 445-448 (1986).
17. Robertson, E.J. in Teratocarcinomas and Embryonic Stem Cells (ed. Robertson, E.J.) 71-112 (IRL, Oxford, 1987).
18. Kim, H.S. & Smithies, O. Nucleic Acids Res. 16, 8887-8903 (1988).
19. Breier, G., Bucan, M., Francke, U., Colberg-Poley, A.M. & Gruss, P. EMBO J.5, 2209-2215 (1986).
20. Joyner, A.L., Skarnes, W.C. & Rossant, J. Nature 338, 153-156 (1989).
21. McKay, R. D. G. Cell 58, 815-821 (1989).

## Revendications

1. Procédé pour obtenir l'expression, ou pour améliorer le taux d'expression d'un gène, dit gène receveur, dans le génome d'une cellule eucaryote, ledit gène receveur étant susceptible d'être rendu fonctionnel ou plus efficace lorsqu'il est recombiné avec un ADN de complément, ledit procédé comprenant :
- l'introduction dans des cellules eucaryotes qui ne sont ni des cellules germinales humaines, ni des cellules embryonnaires humaines, d'un vecteur contenant un insérat comprenant lui-même l'ADN de complément, dit ADN d'insertion, et deux séquences dites "flanquantes" de part et d'autre de l'ADN d'insertion, respectivement homologues à deux séquences génomiques qui jouxtent le site d'insertion souhaité dans le gène receveur
- l'ADN d'insertion comportant une partie d'un gène, et étant hétérologue vis-à-vis du gène receveur ;
- les séquences flanquantes étant choisies afin de permettre par recombinaison homologue, la juxtaposition de l'ADN d'insertion dans le gène receveur, de façon à ce que l'ADN d'insertion rende fonctionnel ou améliore le fonctionnement du gène receveur.

2. Procédé selon la revendication 1 dans lequel l'ADN d'insertion est constitué par une partie d'un gène, par exemple une séquence régulatrice.

3. Procédé selon la revendication 1 dans lequel le gène receveur est un gène qui normalement n'est pas transcrit dans la cellule eucaryote.

4. Procédé selon la revendication 1 dans lequel le gène receveur n'a pas de phénotype sélectionable.

## Claims

1. A method for obtaining the expression or improving the degree of expression of a gene, termed the receiver gene, in the genome of a eukaryotic cell, said receiver gene being capable of being rendered functional or more effective when it is recombined with a complement DNA, said method comprising:
• introducing into eukaryotic cells, which are neither human germinal cells nor human embryonic cells, a vector containing an insert itself comprising the complement DNA, termed insertion DNA, and two sequences termed "flanking" sequences either side of the insertion DNA, respectively homologous to two genomic sequences which adjoin the desired insertion site in the receiver gene;
• the insertion DNA comprising a portion of a gene and being heterologous to the receiver gene;
• the flanking sequences being selected to allow, by homologous recombination, juxtaposition of the insertion DNA in the receiver gene, so that the insertion DNA renders the receiver gene functional or improves its function.

2. A method according to claim 1, in which the insertion DNA is constituted by a portion of a gene, for example a regulatory sequence.

3. A method according to claim 1, in which the receiver gene is a gene which is not normally transcribed in the eukaryotic cell.

4. A method according to claim 1, in which the receiver gene does not have a selectable phenotype.

## Patentansprüche

1. Verfahren zum Erzielen der Expression oder zur Verbesserung der Expressionsrate eines Gens, des sogenannten Empfängergens, im Genom einer eukaryontischen Zelle, wobei das Empfängergen in eine funktionelle Form überführt werden kann oder seine Funktionalität gesteigert werden kann, wenn es mit einer Komplement-DNA rekombiniert wird, wobei das Verfahren umfasst:
- Einführen in die eukaryontischen Zellen, die weder humane Keimzellen noch humane Embryonalzellen sind, eines Vektors, der eine insertion enthält, die ihrerseits die Komplement-DNA, die sogenannte DNA-Insertion, umfasst und zwei sogenannte "flankierende" Sequenzen beiderseits der DNA-Insertion, die jeweils homolog sind zu zwei genomischen Sequenzen, die an die gewünschte Insertionsstelle im Empfängergen angrenzen;
- die DNA-insertion einen Teil eines Gens umfasst und in Bezug auf das Empfängergen heterolog ist;
- die flankierenden Sequenzen so ausgewählt sind, dass sie über eine homologe Rekombination das Eingliedern der DNA-Insertion im Empfängergen ermöglichen, so dass die DNA-Insertion die Überführung des Empfängergens in eine funktionelle Form bewirkt oder dessen Funktionalität steigert.

2. Verfahren nach Anspruch 1, wobei die DNA-Insertion aus einem Teil eines Gens, z.B. einer regulatorischen Sequenz, besteht.

3. Verfahren nach Anspruch 1, wobei das Empfängergen ein Gen ist, das normalerweise nicht in der eukaryontischen Zelle transkribert wird.

4. Verfahren nach Anspruch 1, wobei das Empfängergen keinen selektierbaren Phänotyp aufweist.
